(19)

Europäisches
Patentamt
European
Patent Office
Office européen
des brevets

(11) **EP 3 556 278 A1**

(12) **EUROPEAN PATENT APPLICATION**

(43) Date of publication:
**23.10.2019 Bulletin 2019/43**

(51) Int Cl.:
**A61B 5/00** *(2006.01)*        **A61B 5/05** *(2006.01)*

(21) Application number: **18168515.7**

(22) Date of filing: **20.04.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Valtronic Technologies (Holding) SA
1343 Les Charbonnières (CH)**

(72) Inventor: **Lepple-Wienhues, albrecht
25300 Pontarlier (FR)**

(74) Representative: **Patentanwälte
Ruff, Wilhelm, Beier, Dauster & Partner mbB
Kronenstraße 30
70174 Stuttgart (DE)**

(54) **SCANNING DEVICE FOR LIVING OBJECTS**

(57)     A device for providing a non-contact, time-resolved three-dimensional scan of at least one structural element of a living object which uses microwaves, that comprises besides at least one antenna element, at least one transmitter and at least one receiver, at least one comparator for comparing emitted microwaves and microwaves which are reflected off and at least one analyzer for analyzing at least one property of the emitted microwaves and the received microwaves out of the group of time, frequency, phase, polarization and amplitude.

Further the device comprises at least one control and/or processing unit designed for repetitively sampling two-dimensional images in which each pixel contains information about the precise distance and/or velocity of said structural elements towards the antenna elements.

The invention allows a repeated spatial reconstruction of the structural element in a living object.

Fig. 1

**Description**

**Field of the invention**

The present invention relates to a device for providing a non-contact, time-resolved three-dimensional scan of at least one structural element of a living object. Further, the invention relates to a method for providing data from at least one structural element of a living object using microwaves.

**Background**

Many pathologic conditions of the human body reveal specific sounds or pulsations at specific body locations. These include, but are not limited to: arterial pulse wave velocity related to blood pressure, Arrhythmias, Heart valve stenosis/regurgitations, Aortic stenosis, Arterial stenosis (carotid, etc), Aortic aneurysm, Cardiac insufficiency, Pneumonia, Asthma, Pneumothorax, Sleep apnea, Pleuritis, Inborn heart defects, Gastrointestinal atresias, Ileus (paralytic/stenotic), Hyperperistaltic (enteritis), Fetal heart beat, uterine contractions, Restless legs, Tremor, Paralysis.

Abnormal liquid accumulation in the body is a hallmark of Haematoma (et intracranial), Ascites, Oedema, Pleural effusion, Abscess, Hydrocephalus, and Tumors.

Medical professionals use hearing and tactile examination with their fingers as well as additional contact sensors including stethoscopes, accelerometers, microphones, pressure sensors and the like in order to place the sensors on certain regions of the body and retrieve diagnostic information about sounds, pulsations and/or reflections of sounds and acoustic/subacoustic waves. Using the body location and a broad medical knowledge a diagnose can often be obtained, e.g. by measuring the pulse wave velocity of an arterial pulse traveling from the heart to a peripheral artery, or by hearing a sound typical for an arterial stenosis over the region of the carotid artery or by identifying the typical sound of a pneumonia in the caudal / dorsal regions of the lung. The traditional examination is called auscultation and has been augmented by multiple sensors that can be attached to the skin.

As a consequence, there is a fundamental need for diagnosis by remote, touchless scanning of the body. Several approaches have been proposed to satisfy this need, including MRI (Magnetic Resonance Imaging), Sonography (US), X-Ray Medical Imaging, PET (Positron-Emission Tomography), use of microwaves and others.

**Prior art**

1. Relating to microwaves a recent overview over medical radar applications summarizes the challenges representative for this prior art. The following section (indented) is quoted from R. Chandra, I. Balasingham, H. Zhou, and R.M. Narayanan, "Medical Microwave Imaging and Analysis," Chapter 19 in Medical Image Analysis and Informatics: Computer-aided Diagnosis and Therapy (edited by P.M. de Azevedo-Marques, A. Mencattini, M. Salmeri, and R.M. Rangayyan). Boca Raton, FL: CRC Press, ISBN: 978-1-4987-5139-7, pp. 451-466, 2017.:

These open challenges need to be addressed and more research has to be done before microwave imaging can be effectively used in a real clinical environment. Some of these open challenges are discussed in this section. Coupling the microwave signal to the body: there are large differences in the electrical properties between the air and tissues of the biomedical body. Due to this difference, in the absence of any coupling. or matching medium, microwave signals transmitted from the antenna undergo reflection at the airtissue boundary, leaving a fraction of the transmitted microwave signal to be coupled to the body. The coupled signal is further attenuated by the lossy tissues, leaving a very weak scattered signal to be used for imaging. Hence, matching medium is used to reduce the reflections at the air-tissue boundary. Water (not pure) is usually used as a matching medium. However, water itself has relatively high losses. Therefore, a low-loss matching medium is required. Some research in this regard has been done by Hamsakutty et al. [76], where sodium meta silicate gel for 2.45 GHz imaging frequency is proposed. Another solution for low strength of the scattered signal is to use a high dynamic range system. Contrast agents: In MRI, contrast agents are usually used to enhance the magnetic properties of the tumor that makes it clearly visible in the image. Similar to this, development of a biocompatible contrast agent for microwave imaging of the body is an open challenge. Contrast agents can be used in cases where the difference in the electrical properties of the malignant tissue and the healthy tissue is small enough to be detected by the microwave imaging system. The idea is to administer a contrast agent to the body by methods like intravenous injection, as previously discussed. Some volume of the contrast agent will then reach and bind with the cancerous tissues enhancing their electrical properties. Use of carbon nanotubes and microbubbles as contrast agents for breast cancer has been proposed by Shea et al. [26]. Contrast agents can be specifically used for the qualitative imaging algorithms.

Advancement in the imaging algorithms: At the core of microwave imaging are the imaging algorithms. Computational and memory efficiency are the properties needed for a robust imaging algorithm. Quantitative imaging algorithms used are EM inverse problem and, hence, suffer from open challenges of the inverse problem like nonlinearity and ill-posedness. Usually methods employed to tackle these challenges of an inverse problem result in a computationally and memory demanding imaging algorithms. Thus, there is a scope to develop robust, computational, and memory-effective imaging algorithms. Antennas and Measurement System: Usually, microwave imaging system employs a large number of the antennas in an array. This is done in order to reduce the non-uniqueness and the ill-posedness of nonlinear inverse problems to some extent. However, using a large number of antennas increases the processing complexity of the system and adds to the cost. Moreover, a large number of the antennas means closely spaced antennas that may couple with each other and introduce error in the measured data. Development of an effective antenna system with an optimum number of antennas is a future research direction. A usual measurement system for microwave imaging uses the vector network analyzer (VNA). The VNA-based system records the path-gain between the antennas, rather than the electric-field that is required by the imaging algorithms. To convert the path-gain to the electric field calibration is done. Developing a calibration method that is less prone to errors is also needed. Frequency band and resolution: One of the open challenges of microwave imaging is the usage of an optimum frequency band. The reason for this is that the attenuation of the microwave signal increases at higher frequency. On the other hand, using low attenuation, low frequency bands result in a lower image resolution. Moreover, a frequency band that is optimum for one body part may not be suitable for another part due to variation in the tissue properties and size. Several frequency bands, such as the 403.5 MHz MedRadio band, 900 MHz, UWB (3.1- 10.6 GHz), and so on, have been used, depending upon the application. Thus, a thorough investigation is required before using a particular frequency band for particular application to obtain an acceptable resolution of the image.

2. Prior art disadvantage 1: Existing body scanners (MRI, US, X-ray, PET)

Existing body scanners use x-rays, nuclear magnetic resonance, positron emission or ultrasound in order to retrieve diagnostic information. All these methods have shortcomings. X-rays are ionizing, evoking molecular damage including genetic damage that can lead to cancer. Therefore, the exposure to x-rays must be limited to an absolute strict minimum. Furthermore, x-rays do not well discriminate between different soft tissues and therefore the invasive application of contrasting agents is often required. Nuclear spin magnetic resonance is capable of retrieving images of internal body structures of high spatial resolution. However, the method is slow and unable to resolve movement of organs, e.g. the heartbeat. Furthermore the device complexity is immense and the devices are stationary and very expensive. The same disadvantage is present in positron emission, that requires application of radioactive substances to the patient and has limited spatial resolution. In contrast to these techniques, ultrasound can retrieve diagnostic images with subcentimeter resolution and the devices are inexpensive and increasingly mobile. However, ultrasound is totally reflected at structures with a large acoustic impedance gradient like air or bones leaving blind zones. Furthermore, removal of bedding/ clothing and sensor placement on the body surface with a gel is required to avoid energy reflection at the body surface.

3. Microwave/Radar devices can be used to penetrate clothes, bedding etc. and can penetrate the body to different depths depending on the wavelength used. The reflection is a function of dielectric permittivity of the tissue which varies mainly with the water content, the latter being a function of vascularization and perfusion. Lung tissue consisting mostly of air is almost not reflecting centimeter waves. Fat tissue is poorly reflecting, skin and bone are intermediately reflecting, muscle is a strong reflector and blood or other liquid volumes are almost totally reflecting centimeter waves. Therefore, when directing a centimeter wave beam at the body surface bedding and clothing are penetrated, e.g. in the thorax region the majority of the reflection is observed at the skin surface, and smaller fractions from the sternum bone and from the heart muscle. Almost total reflection is obtained from the heart chamber containing a blood volume.

The dielectric constant of tissue is a function of wavelength, and therefore different penetration characteristics are obtained at different wavelengths. E.g., for security scanners terahertz (i.e. mm) waves do penetrate clothing but are almost totally reflected on the surface of the skin. In contrast, electromagnetic waves in the decimeter band are penetrating through matter including tissue and can be used to detect human bodies underneath debris or a snow layer in emergencies.

4. Prior art disadvantage 2: Existing microwave devices have one dimension only

US20080045832A1 describes a device working with centimeter waves aka microwaves capable of detecting acoustic oscillations of body and tissue surfaces that are representative of heart sounds. The single-dimensional electromagnetic beam is reflected and the oscillations are being recorded without obtaining information of the body area reflecting said beam.

US20160100766A1 describes a device measuring arterial pulse wave velocity using a single-dimensional radar beam to detect the heart contraction and an optical means to detect arterial pulsation in a peripheral artery, thus allowing to calculate the propagation speed of the arterial pulse wave.

5. Prior art disadvantage 3: Existing microwave devices need antennae on skin and water submersion for tomography, extreme computing power required

WO 2017 /125397A1 describes a medical imaging system penetrating the body with microwaves and reconstructing images of cross-sections. To this end, antennas are placed onto the skin with vacuum and a water-based coupling material in order to avoid reflection of the microwaves at the skin or other superficial tissue boundaries and obtain wave scattering information by deep structures, e.g. for detection of tumors in a breast.

6. Prior art disadvantage 4: Existing microwave/radar devices: interferometric SAR (Synthetic Aperture Radar) very slow due to long exposure / processing time

While in this and similar prior art the single-dimensional electromagnetic radar beam can penetrate the body and measure diagnostically relevant acoustic waves emanating from body structures, all of these devices are lacking the crucial information on what segment of the body or what detailed anatomical region said acoustic waves are emitted from. Therefore, using the doctor with the stethoscope in his hand as a reference, only a global body acoustic emission can be obtained and no information about the exact anatomical localization of the acoustic source is obtained from the radar device. This is equivalent to a single microphone in the room to listen to the heartbeat, but not to a doctor's hand strategically locating the stethoscope on different regions of the body.

For geological satellite surveying a radar technique is known that uses the phase shift of reflected electromagnetic waves in order to obtain very fine spatially resolved information about minute changes of elevation of the terrain. While this geological Synthetic Aperture Radar (SAR) Interferometry is useful for tectonic / seismic activity surveillance, the time resolution is limited by the satellite orbit time. The range (distance radar antenna to reflective surface) is typically appr. 800 km. The high lateral resolution is due to the synthetic aperture length being synthesized from individual measurements along the orbital flight path.

Many processing algorithms exist to process SAR data. The standard algorithms are the range-Doppler algorithm, the chirp scaling algorithm, and the Omega-k algorithm. Those algorithms attempt to achieve the resolution and point spread function of an ideal matched filter.

Range resolution is dependent upon the precision of the time-of-flight measurement. This precision can be either increased by shortening the pulse with the drawback of low transmission power levels since power is a function of duration. Alternatively, wider bandwidth requiring long integration times can increase precision and signal-to-noise-ratio.

In ultra-wide-band radar the transmission is code-modulated to achieve a wide bandwidth and range precision. However, as stated before, drawbacks of this approach lie in limited power due to short effective pulse duration as well as in the large frequency band, leading to a poor definition of penetration depth into biological tissues.

In airborne or satellite SAR the reflected waves from different angles at different times can be synthesized into a large synthetic aperture with very high precision. Because the reflected waves need to be analyzed over several hundred milliseconds, this technique works only well for stationary targets as seen in satellite imagery of the ground surface but is not suitable for rapidly moving targets like a vibrating body surface. Furthermore, when surface pixels of a scanned reflection image are moving in opposite ways, the interferometric approach which is based on phase shifts will not be able to resolve range distance changes at high resolution.

7. Prior art Time-Lapse Imaging of Human Heart Motion With Switched Array UWB Radar (IEEE TRANSACTIONS ON BIOMEDICAL CIRCUITS AND SYSTEMS, VOL. 8, NO. 5, OCTOBER 2014)

In this publication a switched array of antenna element pairs was used to obtain a tomographic scan of the thorax region of a subject. By placing the radar antennas in contact with the human body, the strong reflection from the air-body surface was reduced and the radar was able to sense waves that penetrate the tissue and are reflected from the heart wall. An ultra-wideband approach with strong pulse compression was used. The frame rate was 15 Hz or 36 Hz depending on the switch pattern.

The device can resolve movements of the heart wall. However, due to the wide frequency range the penetration depth of the electromagnetic waves was not well defined, since waves at lower frequencies penetrate deeper into the body than waves at higher frequencies. Furthermore, due to the inherent power limitations of the technology used the antenna array had to be in contact with the body to achieve the required signal-to-noise ratio.

**Problem and Solution**

In view of the discussed prior art, it is among others the object of the present invention to develop a new device allowing a non-contact, time-resolved three-dimensional scan of at least one structural element of a living object. With this device data from at least one structural element of a living object should be provided which are useful as diagnostic information concerning a multitude of pathologies.

At least this object is solved by the device with the features of independent claim 1 and by the method with the features of independent claim 10. Preferred embodiments of this device and this method are defined in the dependent claims. The wording of all claims is hereby incorporated into this description by explicit reference.

In the present document we disclose a device using Synthetic Aperture Radar Interferometry related to medicine. This device is suited to create three-dimensional body scans with sufficient lateral and range resolution to obtain body scan images, preferably at a high frame rate, allowing the recording of acoustic and sub-acoustic events in real time and at a high image resolution for the entire body cross-section accessible to the radar/microwave beam.

**Description of the invention**

1. Invention advantage 1: Non-ionizing frequency range (0.5 - 1000 GHz)

The device and method according to the invention are preferably using electromagnetic waves at frequencies ranging from 0.5 GHz to 1000 GHz. Due to the wavelengths these waves do not interfere with submolecular structures and do therefore not create molecular damage in contrast to ionizing radiation. The only known biological effect is heating of tissue, which is not medically relevant at the power levels used in the invention (< 1 W).

2. Invention advantage 2: Fast 3D scan possible with rapid switching SAR

The radar transceiver transmits a modulated electromagnetic wave through a suited antenna. The receiver records a signal reflected by a structural element of the living object, normally an external and/or internal body surface. The transmitted signal is frequency-modulated, amplitude-modulated, or code-modulated so that the timing of the transmission of the signal can be determined a precisely as possible. Then, the range distance is measured by obtaining time of flight of the signal. A movement of a surface of an organ (e.g. left heart chamber) or the skin is detected based on electromagnetic waves reflected by the surface of the biological tissue and/or the acoustically coupled surface of the skin. Since these physiological movements or vibrations can be very small in amplitude, the range distance is measured very precisely by using the phase difference between the transmitted and the reflected signal.

Range resolution

For clarity the subsequent calculations are given as examples for the use of a frequency modulated, continuous wave signal (FMCW) radar. Similar principles apply for other variants of radar, including code-modulated ultra-wideband radar.

In an FMCW radar, after mixing the transmitted and reflected signals the difference signal contains the desired information about the target:

$$f(t) = \cos(\phi(t) - \phi(t - \tau)) = \cos(2\pi(\frac{B\tau}{t_s}t - \frac{B\tau^2}{2t_s} + f_c\tau))$$

$f_c$ is the sweep center frequency, B is sweep bandwidth and $t_s$ is sweep length.

The reflected signal at distance r is received with time of flight delay $\tau = \frac{2r}{c}$ (c speed of light, r range distance). $\frac{B\tau}{t_s}t$ represents the frequency shift due to the FMCW modulation and the term $(B_T^2)/(2t\_s) + f\_c \tau$ represents the phase shift. $\frac{B\tau^2}{2t_s} \approx 0$ because $\tau^2$ is very small. $f_c\tau = f_c\frac{2r}{c} = \frac{2r}{\lambda}$ show effectively the angle resolution of the phase term. The r resolution depends on phase noise and can be estimated at 5.6 GHz ($\lambda$ =54 mm) Assuming that phase detection is <= 1° the minimum detectable range change <= 75 $\mu$m.

Crossrange resolution

$$\delta_{xrange} = \frac{cR}{2fL}$$

c is the propagation speed, f is the frequency, R is the distance between the array and imaging object and L is the effective length of the synthetic array that is twice its physical length.

The synthetic array can be either created by moving the antenna as in the satellite application of by using a two-dimensional array of individual antenna elements.

The synthetic aperture length L is twice the physical aperture length of the antenna array. Using discrete antenna elements in an array, virtual antenna elements can be constructed. E.g., if using 10 discrete transmitter elements and 10 discrete receiver elements, a virtual array of 10 x 10 = 100 individual transceiver elements can be obtained.

Such arrays are known as Multiple Input Multiple Output devices. With such 2D antenna arrays, sparse arrays can reduce the number of active transmitter channels. To minimize the number of active channels required, the resolution can be maximized by optimally choosing the positions of active elements.

The frequencies / wavelengths are chosen in order to obtain a good reflection from surfaces on and inside the body to reveal diagnostic information about important inner organs. Multiple frequency bands can be mixed simultaneously or used sequentially in order to vary depth penetration and information in the reflected signal. The lateral resolution is preferably >1cm, while the range resolution is preferably < 10 $\mu$m. The time resolution is preferably > 10 Hz (>1 kHz, > 5 kHz), corresponding to a frame rate of 10/s (1000, 5000/s).

The range (distance device antenna / body) is preferably 1-5 m.

In a preferred embodiment, the device is used to determine velocity of the arterial pulse wave. To this end, two-dimensional radar scans of the body are taken at a frame rate of 5 fps (1 frame every 200 ms) at a lateral resolution of 1 cm. Using an image area containing the body of 100 x 200 cm, in the resulting images the area resolution is 100 x 200 = 20.000 pixels. Each pixel contains now the precise range distance from the scanner antenna with a distance resolution of 10 $\mu$m. A pattern recognition is applied to detect the outline of the body as well as the heart region and the inguinal region. The pixels laying in both regions are labeled Region of Interest (ROI), ROI[heart] and ROI[femoral artery]. For each ROI a vibration curve depicting range distance over time is calculated. The arterial pulse in the ROI[femoral artery] arrives delayed against the pulse in the ROI[heart], reflecting the arterial pulse wave travel time. Using the distance between the two regions the arterial pulse wave velocity can be determined.

3. Invention advantage 3: Acoustic coupling of physiological vibration to skin surface or tissue close to skin

Surprisingly, it is not required according to the invention to penetrate the body deeply with the electromagnetic waves and analyze complex scattering information from deep tissue layers. Rather, many physiological movements of organs that are altered in disease are acoustically coupled to the surface of the body and/or to tissue surfaces close to the skin. Therefore, the analysis of the bulk reflection coming from superficial tissues allows to retrieve valid information about a wealth of potentially pathologic conditions without the need for deep penetration of the emitted microwaves into the body or the complex analysis of small energies scattered deep inside the body.

The inventive device can provide data relevant for a variety of pathologies. In the subsequent preferred embodiments only some examples are given in order to explain the function principles. It is obvious to a person skilled in the art that those principles apply to a wealth of pathologic conditions that lead to abnormal acoustic patterns in tissue and body surfaces.

In a preferred embodiment the scanned images are analyzed automatically. The contours in the living object, e.g. the human body and/or the posture and/or orientation of this body are analyzed and the body image is segmented into anatomically relevant segments. The corresponding pixels for a relevant anatomical segment are analyzed acoustically. The acoustic analysis includes frequency spectrum and sound patterns. For example, a pneumonia typically results in crackling or bubbling noises synchronous with inhaling. The thoracic surface visible to the device is segmented and the segments vibrations and/or acoustic patterns are analyzed for the presence of suspicious sounds and noises.

In a preferred embodiment the acoustic data provided according to the invention can be stored in a database and correlated with the clinical diagnose, such enabling the system to compare the patient data with stored data and pattern of stored data typical for a certain disease. A self-learning algorithm can be used in order to increase diagnostic accuracy.

In a preferred embodiment the neck region is automatically located in the scanned images and the acoustic analysis is performed in order to detect the typical sounds of stenosis of the carotid artery. Since the arterial pulse timing can be obtained from pixels representative of the heart region the pulse-synchronous sounds resulting from turbulent blood flow can be detected (carotid bruit) and analyzed.

In a preferred embodiment the body contours and/or the posture and/or orientation of the body are analyzed automatically and the musculature of the face and the extremities is analyzed for spontaneous movements. The analysis emphasizes on asymmetric movement for stroke detection typically including hemiplegic paralysis by unilateral damage to motoneurons. Other neurologic disorders can be detected in a similar fashion, e.g. restless leg syndrome, tremors and others.

In a preferred embodiment the abdominal surface of a pregnant woman can be scanned in order to record uterine contractions as well as the fetal heartbeat that is acoustically coupled to the abdominal surface. Thus, the device can be used for touchless tocographic monitoring.

In a preferred embodiment the device is used in a clinical environment, where the patient is placed by a medical professional in any body posture and is asked to change body posture and orientation in order to allow for a complete scan of relevant body surfaces.

In a preferred embodiment the device is used in an ambulatory and/or home environment, allowing for monitoring of body suface movements and vibrations. For example the device can be used for sleep monitoring, where respiratory movements and sounds are being monitored as well as cardiovascular parameters including pulse wave velocity, pulse frequency and regularity. Furthermore, spontaneous movements and movements of the eyes are being monitored (REM phase sleep). Thus, pathologic conditions like sleep apnoea can be assessed.

4. Invention advantage 4: SAR with multiple transmitting / receiving elements and/or rapid movement for short exposure time

In the prior art the synthetic aperture retrieves crossrange resolution information based on triangulation measurements, i.e. scanning details in a lateral and vertical dimension. Since the direct line of sight from the antennae to the object is called "range", this information is known as "crossrange resolution". In order to obtain a large synthetic aperture, and thus a high crossrange resolution, the triangulations are performed mostly sequentially, using one antenna pair at a time. Therefore, the possibility to obtain a high frame rate is limited, and acoustic movements of surfaces can not be timely resolved.

In a preferred embodiment, means are implemented that allow for either a fast sequential performance or a parallel performance of said triangulations, thus allowing a large aperture yielding a high crossrange resolution at a high framerate. E.g., for scanning a horizontal line of pixels across a body, the antenna array is rapidly switched between elements. Different elements use different beam polarization, allowing for simultaneous triangulation at different antenna elements while minimizing crosstalk. Different elements use adjacent frequency bands, allowing for simultaneous triangulation at different antenna elements while minimizing crosstalk. Different elements use different frequency pattern or code pattern modulations, allowing for simultaneous triangulation at different antenna elements while minimizing crosstalk. The analyzer is using time, amplitude, phase, frequency, code and polarization information in order to perform triangulations at different body locations in a rapid, repeated fashion. E.g., a given transmitter/receiver antenna pair is using a frequency band of 200 MHz with a center frequency of 5.1 GHz while a separate transmitter/receiver antenna pair is using a frequency band of 200 MHz with a center frequency of 5.2 GHz at vertical polarization. Another transmitter/receiver antenna pair is using the same frequency band of 200 MHz with a center frequency of 5.2 GHz but is using horizontal polarization. By assigning different frequency bands and/or polarization orientation to different transmitter/receiver antenna pairs spatially resolved signals can be acquired simultaneously and therefore more rapidly, allowing for a higher frame rate. Surprisingly, by using these means a frame rate of > 50 Hz, >500 Hz, and even >5 kHz can be achieved at a lateral resolution of $\leq$ 10 mm. The range resolution is better than 10 $\mu$m.

In a preferred embodiment, the contours and anatomical patterns of the body as well as their respective position are being determined and analyzed automatically.

In another embodiment the scan is then restricted to anatomical areas of interest, in order to allow for more rapid scanning repeats and thus for a higher frame rate in order to resolve higher acoustic frequencies of interest. E.g., for an analysis of heart sounds, the central thoracic region is recognized and located automatically and the corresponding pixels are identified. For a given time period the scan is selectively restricted to said thoracic region, instead of scanning the entire body area. Therefore, the scanning frame rate and the time resolution are increased and vibrations of tissue surfaces can be resolved at high sampling rates, allowing for analysis of higher acoustic frequencies. The analysis algorithm can then switch at certain time intervals between separate anatomical regions of interest. E.g. the algorithm can first focus on the heart area for heart sounds, later on the neck area for carotid artery and breathing sounds, then on the caudal areas of the lung for breathing sounds, then on the inguinal region for femoral artery sounds, and so forth, resembling a doctor placing the stethoscope on different body regions.

In a preferred embodiment, an array of transmitter/receiver antenna pairs is connected to multiple transmitters/receivers allowing for simultaneous emission/reception of electromagnetic waves.

In another preferred embodiment, an array of transmitter/receiver antenna pairs is connected to at least one transmitter/receiver by radio frequency switches, allowing for rapid switching of said antennas and sequential emission/reception of electromagnetic waves.

In another preferred embodiment, an array of transmitter/receiver antenna pairs is connected to at least one transmitter/receiver by radio frequency switches, allowing for a combination of simultaneous and sequential emission/reception of electromagnetic waves.

In another preferred embodiment, at least one transmitter/receiver antenna pair is moved along a defined trajectory and emission/reception of electromagnetic waves at different known locations is used to synthesize an enlarged aperture, multiplying the information received in order to triangulate the position of a reflective surface element.

In a preferred embodiment, a narrow bandwidth is used to define the penetration depth into the body. Preferably a frequency bandwidth is used that penetrates only a few centimeters into the body, so that the waves are reflected mainly from the body surface and from structures close to the surface with strong permittivity gradients, e.g. the anterior heart chamber wall and large superficial arteries.

In a preferred embodiment, a switched array of antenna elements is used where a narrower bandwidth allows for a faster switching rate between antenna elements.

Further advantages and features of the invention will become clear from the following description of the drawings. The individual features can be realized either singly or jointly in combination in one embodiment of the invention. The drawings only serve for illustration and better understanding of the invention and are not to be understood as in anyway limiting the invention.

The drawings schematically show:

Fig. 1: A block diagram of the inventive device,

Fig. 2: An embodiment of the inventive method, which can be performed with an inventive device,

Fig. 3: An alternative embodiment of the inventive method, and

Fig. 4: A representation which explains how data are provided according to the invention.

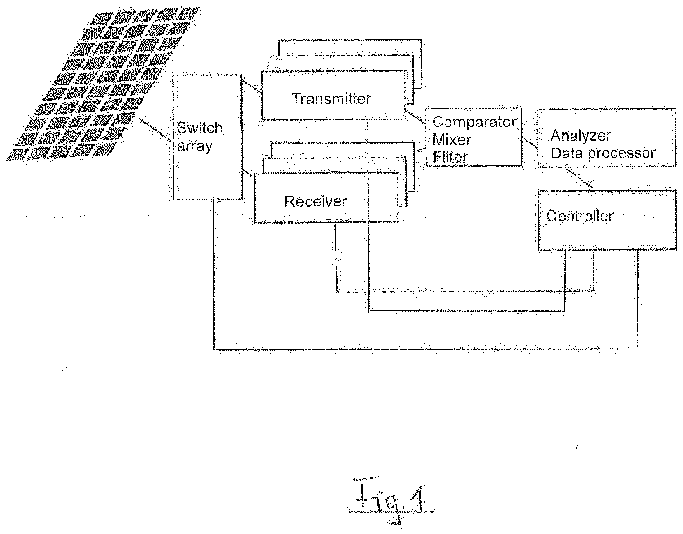

Fig.1 shows a block diagram of the inventive device. In this representation the main components of the inventive device and their arrangement and connection is schematically shown.

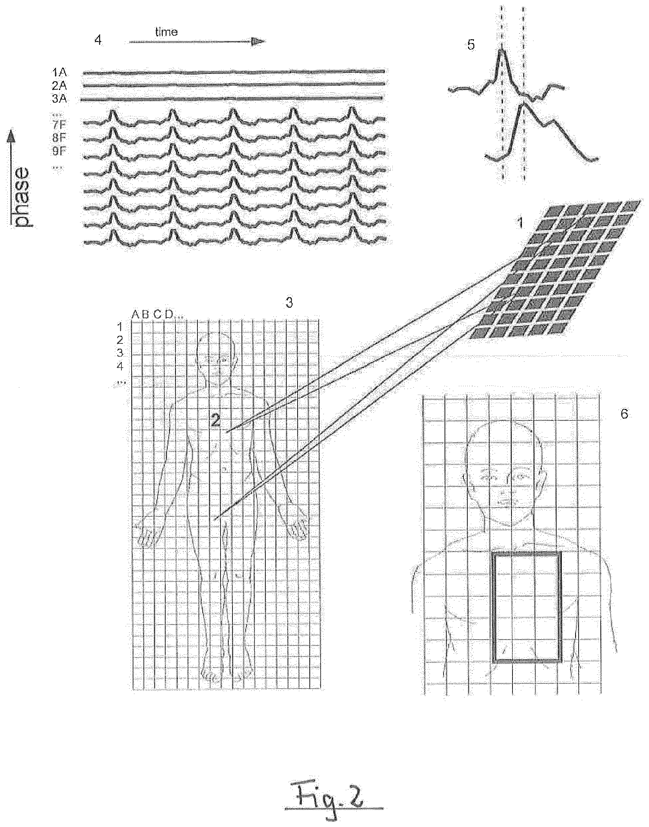

Fig. 2 shows an antenna array 1, sending and receiving waves to and from a body 2, scanning the body surface area in a vertical and horizontal resolution indicated by the 2D pixel grid 3. For each 2D pixel then the phase is analyzed to achieve a high range resolution when body surface and tissue areas move or vibrate. For each 2D pixel a range movement information over time 4 is extracted allowing for vibration analysis on anatomic regions. An example is given for two pixels allowing for determination of arterial pulse wave velocity 5. The upper trace represents a precardiac pixel, the lower trace an inguinal pixel. By analysis of the respective pulse waves and their phase shift, including the 2D distance between the pixels, an arterial pulse wave velocity can be calculated. In another example 6, the scan is temporarily restricted to the precardiac area in order to allow for higher scan rates and higher frequency resolution.

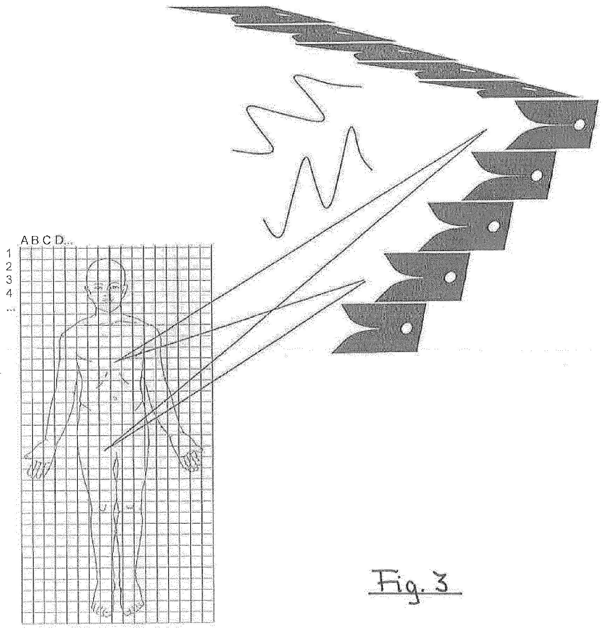

Fig. 3 shows an array (not all elements shown) of highly polarizing vivaldi microwave antennas. In this example the polarization is offset by 90° for vertical versus horizontal scan.

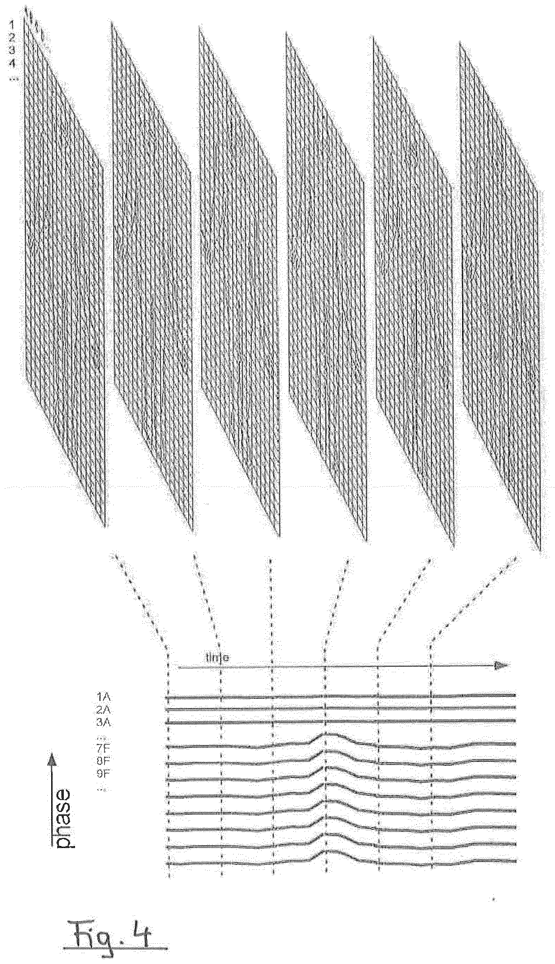

Fig. 4 shows how the acoustic / phonographic information is retrieved for each pixel in the 2D scan using subsequent frames at a high frame rate.

Fig. 4 explains how the imaging of the corresponding structural element of a living object (repeated spatial reconstruction) is provided according to the invention.

**Claims**

1. Device for providing a non-contact, time-resolved three-dimensional scan of at least one structural element of a living object, using microwaves, preferably microwaves with frequencies between 0.5 GHz to 1000 GHz, comprising

   - at least one antenna or antenna element,
   - at least one transmitter for emitting microwaves,
   - at least one receiver for receiving microwaves,
   - at least one comparator for comparing emitted microwaves and received microwaves, reflected off said at least one structural element,
   - at least one analyzer for analyzing at least one property of the emitted microwaves and the received microwaves out of the group of time, frequency, phase, polarization and amplitude, and
   - at least one control and/or processing unit designed for repetitively sampling from said compared and analyzed microwaves two-dimensional images in which each pixel contains information about the precise distance R and/or velocity v of said structural element towards said at least one antenna or antenna element, wherein for each pixel of said images the functions over the time of distance R(t) and/or velocity v(t) can be calculated, resulting in a repeated spatial reconstruction of said structural element of said living object.

2. Device according to claim 1, wherein said unit is designed for a repetition rate of the image scanning of $\geq$ 5 Hz (Hertz), in particular $\geq$ 50 Hz, wherein preferably said repetition rate is $\geq$ 500 Hz or $\geq$ 1000 Hz or $\geq$ 5000 Hz.

3. Device according to claim 1 or claim 2, having an aperture obtaining a resolution $\leq$ 1 cm and/or $\leq$ 1 mm perpendicular to the orientation of the line of sight between the at least one antenna or antenna element and the object.

4. Device according to any one of the preceding claims, where the aperture is a synthetic aperture obtainable by movement of at least one antenna or antenna element or by at least one array of multiple antennas or antenna elements.

5. Device according to any one of the preceding claims, wherein multiple antennas or antenna elements are connected to at least one receiver and /or at least one transmitter, preferably by switches.

6. Device according to any one of the preceding claims, wherein said microwaves are modulated using frequency and/or code patterns.

7. Device according to any one of the preceding claims, wherein said at least one transmitter and/or said at least one receiver contains at least one MASER (Microwave Amplification by Stimulated Emission of Radiation).

8. Device according to any one of the preceding claims, wherein said at least one comparator comprises at least one electronic mixer and/or at least one electronic filter.

9. Device according to any one of the preceding claims, wherein said at least one analyzer and/or said at least one unit is using fast Fourier transformation to analyze information of the transmitted and received microwaves.

10. Method for providing data from at least one structural element of a living object using microwaves, preferably microwaves with frequencies between 0.5 GHz to 1000 GHz, wherein

   - microwaves are emitted from a transmitter in direction to a remote living object via an antenna or antenna element,
   - microwaves reflected off said living object are received by a receiver via an antenna or antenna element,
   - emitted microwaves and received microwaves are compared in a comparator,
   - at least one property of the emitted microwaves and the received microwaves out of the group of time, frequency, phase, polarization and amplitude is analyzed in an analyzer, and
   - in a control and/or processing unit two-dimensional images from said compared and analyzed microwaves are repetitively sampled, wherein each pixel of said images contains information about the precise distance R and/or velocity v of said structural element towards said at least one antenna or antenna element, wherein for each pixel of said images the functions over the time of distance R(t) and/or velocity v(t) can be calculated, resulting in a repeated spatial reconstruction of said structural element of said living object.

11. Method according to claim 10, further **characterized by** at least one of the features defined in claims 2 to 9.

12. Method according to claim 10 or claim 11, wherein the device according to anyone of claims 1 to 9 is used for performing the method.

13. Method according to any one of claims 10 to 12, wherein the reconstruction of a structural element of a living object provided is used in the diagnosis of at least one medical condition or disease.

Fig.1

Fig.2

Fig. 3

Fig.4

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GHASR MOHAMMAD TAYEB ET AL: "Wideband Microwave Camera for Real-Time 3-D Imaging", IEEE TRANSACTIONS ON ANTENNAS AND PROPAGATION, IEEE SERVICE CENTER, PISCATAWAY, NJ, US, vol. 65, no. 1, 1 January 2017 (2017-01-01), pages 258-268, XP011638422, ISSN: 0018-926X, DOI: 10.1109/TAP.2016.2630598 [retrieved on 2017-01-02] * the whole document * | 1-13 | INV. A61B5/00 A61B5/05 |
| X | SHERIF SAYED AHMED ET AL: "A Novel Fully Electronic Active Real-Time Imager Based on a Planar Multistatic Sparse Array", IEEE TRANSACTIONS ON MICROWAVE THEORY AND TECHNIQUES, PLENUM, USA, vol. 59, no. 12, 1 December 2011 (2011-12-01), pages 3567-3576, XP011389183, ISSN: 0018-9480, DOI: 10.1109/TMTT.2011.2172812 * the whole document * | 1-13 | |
| X | WO 2014/196864 A1 (TNO [NL]) 11 December 2014 (2014-12-11) * the whole document * | 1-13 | TECHNICAL FIELDS SEARCHED (IPC) A61B |
| A | US 2005/116947 A1 (LOVBERG JOHN [US] ET AL) 2 June 2005 (2005-06-02) * abstract * | 1-13 | |

The present search report has been drawn up for all claims

| Place of search | Date of completion of the search | Examiner |
|---|---|---|
| The Hague | 13 September 2018 | Van Dop, Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 16 8515

This annex lists the patent family members relating to the patent documents cited in  the above-mentioned European search report.
The members are as contained in the European Patent Office EDP file on
The European Patent Office is in no way liable for these particulars which are merely given for the purpose of information.

13-09-2018

| Patent document cited in search report | | Publication date | Patent family member(s) | | Publication date |
|---|---|---|---|---|---|
| WO 2014196864 | A1 | 11-12-2014 | EP<br>US<br>WO | 3003134 A1<br>2016135694 A1<br>2014196864 A1 | 13-04-2016<br>19-05-2016<br>11-12-2014 |
| US 2005116947 | A1 | 02-06-2005 | NONE | | |

EPO FORM P0459

For more details about this annex : see Official Journal of the European Patent Office, No. 12/82

**EP 3 556 278 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080045832 A1
- US 20160100766 A1
- WO 2017125397 A1

### Non-patent literature cited in the description

- Medical Microwave Imaging and Analysis. **R. CHANDRA ; I. BALASINGHAM ; H. ZHOU ; R.M. NARAYANAN.** Medical Image Analysis and Informatics: Computer-aided Diagnosis and Therapy. CRC Press, 2017, 451-466
- *IEEE TRANSACTIONS ON BIOMEDICAL CIRCUITS AND SYSTEMS,* October 2014, vol. 8 (5